# EUROPÄISCHE PATENTSCHRIFT

(11) **EP 2 850 388 B1**
(45) Veröffentlichungstag und Bekanntmachung des Hinweises auf die Patenterteilung: **06.06.2018**
(21) Anmeldenummer: 13724794.6
(22) Anmeldetag: 17.05.2013
(51) Int. Cl.: A61B 34/20, G01C 11/08, G01C 11/12, G01C 11/14, G01C 3/08

(54) **REGISTRIERVERFAHREN UND -VORRICHTUNG FÜR EIN POSITIONSERFASSUNGSSYSTEM**
REGISTRATION METHOD AND REGISTRATION DEVICE FOR A POSITION DETECTION SYSTEM
PROCÉDÉ ET DISPOSITIF D'ENREGISTREMENT POUR UN SYSTÈME DE DÉTECTION DE POSITION

(30) Priorität: 18.05.2012 DE 102012208389
(43) Veröffentlichungstag der Anmeldung: 25.03.2015
(73) Patentinhaber: Fiagon GmbH, 16761 Hennigsdorf (DE)
(72) Erfinder: KRÜGER, Timo, 13465 Berlin (DE); MUCHA, Dirk, 13467 Berlin (DE); ROSE, Andreas, 16727 Oberkrämer (DE)
(74) Vertreter: Eisenführ Speiser
(86) Internationale Anmeldenummer: PCT/EP2013/060299
(87) Internationale Veröffentlichungsnummer: WO 2013/171338

(56) Entgegenhaltungen:
- WO-A1-2011/134083
- WO-A1-2012/056034
- DE-A1-102009 030 731

## Beschreibung

Die Erfindung betrifft ein Registrierverfahren zum Erfassen der Position und Ausrichtung eines Objekts oder Körperteils in Bezug auf ein Positionserfassungssystem. Die Erfindung betrifft außerdem eine Vorrichtung zum Durchführen eins solchen Verfahrens.

Positionserfassungssysteme, die beispielsweise im medizinischen Bereich eine Navigation von Instrumenten, beispielsweise chirurgischer Instrumente, unterstützen, sind grundsätzlich bekannt. Solche Positionserfassungssysteme können optische, Ultraschallgestützte oder elektromagnetische Positionserfassungssysteme sein. So sind beispielsweise elektromagnetische Positionserfassungssysteme bekannt, bei denen ein Feldgenerator ein elektromagnetisches Wechselfeld erzeugt und Positionssensoren vorgesehen sind, die Spulen aufweisen. Durch das elektromagnetische Wechselfeld des Generators werden in den Spulen Ströme induziert, die von der Ausrichtung einer jeweiligen Spule zum elektromagnetischen Wechselfeld abhängen. Wenn ein bewegliches Instrument mit derartigen Positionssensoren in Form von Sensorspulen ausgerichtet ist, ist es möglich, Ort und Lage des Instruments relativ zu einem Referenzsensor, der beispielsweise ebenfalls Spulen aufweisen kann, zu bestimmen. Vorzugsweise ist hierbei der Referenzsensor als Patientenlokalisator fest mit einem Körperteil eines Patienten (oder auch einem anderen Objekt) verbunden.

Für eine Navigation relativ zu Körperteilen eines Patienten wird typischerweise die Position eines Instrumentes mit einem derartigen Positionserfassungssystem erfasst und die Position des Instrumentes in tomographisch gewonnenen Schnittbildern des Körperteils angezeigt. Damit dies funktioniert, müssen die Positionswerte, die der Positionssensor des Instrumentes liefert, in Koordinaten des tomographischen Abbildes des Patienten übertragen werden. Dazu ist es beispielsweise bekannt, aus einem tomographischen Abbild eines Patienten ein topographisches Abbild der Oberfläche eines Körperteils zu generieren, um Punkte auf der Oberfläche des topographischen Abbilds (im Folgenden auch Modelloberfläche genannt) solchen Punkten auf der Oberfläche des realen Körperteils zuzuordnen, die jeweils von einem Zeige- oder Abtastinstrument berührt werden. So kann im Rahmen eines Registrierverfahrens eine Transformationsvorschrift für mittels des Positionserfassungssystems erfasste Positionswerte in Modellkoordinaten erstellt werden. Dazu wird auf der realen Oberfläche des Körperteils eine Mehrzahl von Punkten abgetastet und die zugehörigen Positionswerte, die ja Punkte auf einer realen Oberfläche repräsentieren, werden Punkten auf der Modelloberfläche unter Beibehaltung ihrer relativen Position zueinander so zugeordnet, dass sich ein möglichst kleiner Fehler ergibt. Hieraus ergibt sich eine Transformationsvorschrift, die angibt, wie erfasste Positionswerte in Koordinaten des topographischen Abbildes - hier auch als topographisches Modell bezeichnet - und damit auch in Koordinaten des tomographischen Abbildes oder Modells, umzurechnen sind.

Die Patientenregistrierung bezeichnet das Ermitteln einer Transformationsfunktion zum Übereinbringen intraoperativ erfasster Positionsdaten mit Positionsinformationen in präoperativ, beispielsweise tomografisch, gewonnener Bilddaten. Für die Patientenregistrierung wird beispielsweise, wie oben beschrieben, ein Patientenmodell erfasst und eine Transformationsfunktion ermittelt, die im Rahmen des Registrierverfahrens erfasste Positionsdaten und ein daraus resultierendes Patientenmodell mit Positionsinformationen zu den präoperativ gewonnenen Bilddaten übereinbringt.

Für die Bestimmung der Transformationsfunktion werden gleiche geometrische Merkmale des Modells und der (beispielsweise tomografisch gewonnenen) Bilddaten in dem jeweiligen Koordinatensystem ermittelt. Über diese Merkmale erfolgt dann die Zuordnung beider Koordinatensysteme. Allgemein verbreitet ist die Oberflächenregistrierung mittels eines Zeigerinstrumentes. Dabei dient als korrespondierendes Merkmal die Hautoberfläche des Patienten. Intraoperativ wird mit einem Zeigeinstrument die Hautoberfläche abgetastet und mit der extrahierten Hautoberfläche aus den Bilddaten in Übereinstimmung gebracht.

WO 2011/134083 A1 offenbart eine Vorrichtung zum Durchführen eines Registrierverfahrens, wobei die Vorrichtung ein Positionserfassungssystem mit einer Referenzmarkenanordnung sowie eine Bildsensoreinheit aufweist.

Ein Vorrichtung zur Darstellung von Bilddaten, welche ein optisches Gerät, eine Lageerfassungseinrichtung zum Erfassen der Lage des optischen Gerätes, eine mit dem optischen Gerät und der Lageerfassungseinrichtung verbundene Bilddatenverarbeitungseinrichtung und eine mit der Bilddatenverarbeitungsvorrichtung verbundene Anzeigeeinheit aufweist ist in der WO 2012/056034 A1 beschrieben. Die Lageerfassungseinrichtung weist einen Feldgenerator und eine Sensorspule auf.

Der Erfindung liegt die Aufgabe zugrunde, ein möglichst einfach und schnell durchführbares Registrierverfahren anzugeben.

Erfindungsgemäß wird diese Aufgabe durch ein Registrierverfahren zum Erfassen der Position und Ausrichtung eines Objekts oder Körperteils in Bezug auf ein elektromagnetisches Positionserfassungssystem gelöst, wobei das Verfahren die folgenden Schritte umfasst:
- Anordnen eines Referenzsensors an dem Objekt oder Körperteil, derart, dass der Referenzsensor optisch von einer Bildsensoreinheit erfasst werden kann, und zwar so, dass seine Relativposition zu einer zu erfassenden Oberfläche des Objekts oder Körperteils aus optisch erfassten Informationen ermittelbar ist,
- Erfassen der Position des Referenzsensors mittels des elektromagnetischen Positionserfassungssystems,
- fotogrammetrisches Erfassen einer Oberfläche des Objektes oder Körperteils und des Referenzsensors mittels einer optischen monofokalen Bildsensoreinheit,
- Erzeugen eines Oberflächenmodells des Objektes oder Köperteils basierend auf fotogrammetrisch erfassten Informationen,
- Bestimmen der Position des Referenzsensors in dem Oberflächenmodell und
- Zuordnen des Oberflächenmodells zu einem Koordinatensystem des elektromagnetischen Positionserfassungssystems anhand von der bestimmten Position des Referenzsensors in dem Oberflächenmodell und der mittels des elektromagnetischen Positionserfassungssystems erfassten Position des Referenzsensors.

Das erfindungsgemäße Registrierverfahren bietet den Vorteil, dass es eine berührungslose Registrierung erlaubt und damit Probleme vermeidet, die sich bei bekannten Registrierverfahren mittels Zeigerinstrument beispielsweise daraus ergeben, dass sich weiche Haut eines Patienten bei Berührung mit dem Zeigerinstrument verformen kann. Da mit der optischen Registrierung außerdem ein größerer Teil der Oberfläche eines Köperteils oder Objekts gleichzeitig erfasst werden kann, kann die Registrierung auch schneller und gleichzeitig genauer erfolgen. Das fotogrammetrische Erfassen einer Oberfläche des Objektes oder Körperteils erfolgt mittels einer optischen, monofokalen, Bildsensoreinheit, die vorzugsweise mit einem Positionssensor verbunden ist oder einen solchen aufweist, dessen Position mittels des Positionserfassungssystems erfassbar ist. Dies erlaubt es, die Bildsensoreinheit während der Registrierung, aber auch später, relativ zum Körperteil oder Objekt sowie relativ zum Positionserfassungssystem zu bewegen, um so den Köperteil oder das Objekt aus mehreren oder der jeweils günstigsten Lage optisch erfassen zu können. Falls die Bildsensoreinheit mit einem Positionssensor ausgestattet ist, kann dieser als Referenzsensor dienen und so einen am Körperteil oder Objekt angeordneten Referenzsensor ersetzen oder ergänzen. Der Positionssensor der Bildsensoreinheit liefert in diesem Fall Informationen zur Lage (und Ausrichtung) der Bildsensoreinheit. Erfindungsgemäß wird ein Referenzsensor genutzt, der optisch von der Bildsensoreinheit erfasst werden kann, uns zwar so, dass seine Relativposition zu der zu erfassenden Oberfläche aus den optisch erfassten Informationen ermittelbar ist.

Es wird nun ein Verfahren zur kontaktlosen Patientenregistrierung mittels optischem monofokalen Bildsensor beschrieben. Vorzugsweise wird die Lage (und Ausrichtung) der Bildsensoreinheit relativ zum Patienten überwacht. Dies geschieht mit einem Messsystem, vorzugsweise dem Positionserfassungssystem. Weiterhin sind vorzugsweise die Abbildungseigenschaften der optischen Bildsensoreinheit von vornherein bekannt.

Gemäß einer bevorzugten Ausführungsvariante des Registrierverfahrens wird die Bildsensoreinheit beim Registriervorgang kontinuierlich über die zu erfassende Oberfläche geführt. Dabei werden die relative Position der Bildsensoreinheit zum Patienten (genauer: zum Positionserfassungssystem) - also die Lage der Bildsensoreinheit - und die fotogrammetrisch erfassten Informationen (d.h. in der Regel optisch erfasste Bilddaten) aufgenommen.

Vorzugsweise erfolgt das fotogrammetrische Erfassen der Oberfläche unter Verwendung natürlicher Beleuchtung, d.h. am Ort vorhandener Beleuchtung.

Die Lageinformation und die optisch erfassten Bilddaten werden vorzugsweise einem iterativen Algorithmus zugeführt. Dieser Algorithmus detektiert in den optisch erfassten Bilddaten (z.B. in Einzelbildern) Objekte (Kanten, Linien, Kreise, etc.) und kann diese auch über verschiedene Einzelbilder hinweg einander zuordnen. Es entsteht somit eine Liste von Objekten, die aus verschiedenen Betrachtungsrichtungen und -positionen von der Bildsensoreinheit aufgenommen wurden.

Vorzugsweise kommt anschließend ein weiterer Algorithmus zur Anwendung, der aus diesen Daten die räumliche Position der Objekte ermitteln kann. Dazu muss ein Objekt in mindestens zwei verschiedenen Betrachtungsrichtungen und -positionen aufgenommen worden sein. Dann kann über Triangulierung die Objektposition ermittelt werden.

Werden genügend Objekte und deren Position ermittelt, kann eine herkömmliche Oberflächenregistrierung durchgeführt werden.

Alternativ kann das Registrierverfahren unter Verwendung künstlicher Beleuchtung auch wie folgt durchgeführt werden:
Gemäß einer solchen alternativen Ausführungsvariante ist die Bildsensoreinheit (vorzugsweise starr) mit einem Musterprojektor verbunden. Die relative Position von Bildsensoreinheit zu Musterprojektor und die Abbildungseigenschaften des Musterprojektors sind vorzugweise von vornherein bekannt. Alternativ kann der Musterprojektor auch mit einem Positionssensor verbunden sein, so dass die relative Position von Bildsensoreinheit zu Musterprojektor zu jedem Zeitpunkt aus den Positionsdaten der Positionssensoren des Musterprojektors und der Bildsensoreinheit bestimmt werden kann. Dann kann der Musterprojektor vorteilhafterweise unabhängig von der Bildsensoreinheit bewegt werden, so dass die Bildsensoreinheit jeweils besonders aussagekräftige Einzelbilder mit gut auswertbarer Musterverzerrung aufnehmen kann.

Mittels des Musterprojektors kann im Aufnahmebereich der Bildsensoreinheit auf der Zieloberfläche eine künstliche Struktur (ein Muster, zum Beispiel ein Streifenmuster) projiziert werden, deren Maße bekannt sind. Das auf die Oberfläche projizierte Muster wird von der Bildsensoreinheit optisch erfasst, so dass sich Einzelbilder ergeben, die das auf die Oberfläche projizierte Muster mit seinen die Form der Oberfläche bedingten Verzerrungen zeigen. Anhand der Verzerrungen des Musters kann die dreidimensionale Oberfläche in jedem Einzelbild ermittelt werden. Durch das Zusammenführen der dreidimensionalen Teiloberflächen der Einzelbilder anhand der überlappenden Bereiche kann eine Gesamtoberfläche bestimmt werden. Diese kann dann mit der herkömmlichen Oberflächenregistrierung verwendet werden.

Der Musterprojektor kann ausgebildet sein, das Muster mit Infrarotlicht oder ultraviolettem Licht zu projizieren. In diesem Fall ist die Bildsensoreinheit vorzugsweise mit einem infrarot- oder ultraviolett-empfindlichen Bildsensor ausgestattet. Diese Ausführungsvariante hat den Vorteil, dass das projizierte Muster für einen Operateur nicht sichtbar ist und daher auch nicht stören kann.

Anstelle von oder zusätzlich zum Musterprojektor kann auch eine selbstklebende Musterfolie vorgesehen sein. Diese Folie wird im Zielgebiet befestigt. Durch die bekannten Muster auf der Folie kann die Form der Folie und somit auch die Oberflächenstruktur detektiert werden.

Eine weitere Alternative des Registrierverfahrens sieht die Verwendung einer 3D-Kamera als Bildsensoreinheit vor, die die zu erfassende Oberfläche von vornherein dreidimensional aufnehmen kann. Es erübrigt sich dann die Rekonstruktion der Oberflächenform aus zweidimensionalen Bilddaten, wie sie bei den zuvor genannten Verfahrensvarianten nötig ist. Bekannte 3D-Kameras nehmen eine dreidimensionale Oberflächenform auf, indem die Laufzeit von Infrarotlichtimpulsen gemessen wird. Die Art der Aufnahme ist auch als TOF (time of flight) Methode bekannt.

Um bei allen Ausführungsvarianten des Registrierverfahrens mögliche Verzerrungen eines elektromagnetischen Wechselfeldes des Positionserfassungssystems bestimmen zu können, ist es bevorzugt, wenn mehrere Positionssensoren an unterschiedlichen Orten verwendet werden oder ein beweglicher Positionssensor verwendet wird, der bei dem fotogrammetrischen Erfassen einer Oberfläche des Objektes oder Körperteils bewegt wird und deren bzw. dessen Position ebenfalls fotogrammetrisch erfasst wird. Die Verzerrungen des elektromagnetischen Wechselfeldes können dann aus dem jeweils fotogrammetrisch erfassten Ort des beweglichen Positionssensors oder der Positionssensoren sowie der von dem jeweiligen Positionssensor selbst mittels des Positionserfassungssystems bestimmen Ort ermittelt werden. Es kann dann also sozusagen das elektromagnetische Wechselfeld selbst fotogrammetrisch vermessen werden.

Die Bildsensoreinheit kann eine kleine Kamera oder ein Mikroskop sein.

Eine kontinuierliche Registrierung kann vorteilhafter Weise so durchgeführt werden, dass kein relativ zum Körperteil oder Objekt ortsfester Referenzsensor mehr benötigt wird. Vielmehr würde ein Positionssensor am jeweiligen Instrument und/oder einer beweglichen Bildsensoreinheit die Funktion in einem jeweiligen Zeitpunkt übernehmen. Ein solches Instrument bzw. eine solche Bildsensoreinheit kann beispielsweise ein Mikroskop sein, das mit einem Positionssensor ausgestattet ist.

Die Erfindung soll nun anhand eines Ausführungsbeispiels mit Hilfe der Figuren näher erläutert werden. Von den Figuren zeigt:
- Fig. 1:: Ein Beispiel einer Vorrichtung zum Durchführen eines Registrierverfahrens zum Erfassen der Position und Ausrichtung eines Objekts oder Körperteils in Bezug auf ein Positionserfassungssystem, wobei die Vorrichtung neben einem Positionserfassungssystem einen an der zu erfassenden Oberfläche angeordneten Positionssensor und eine Bildsensoreinheit in Form einer optischen Kamera aufweist;
- Fig. 2:: eine nicht beanspruchte Variante der Vorrichtung aus Figur 1, bei der der Positionssensor nicht an der zu erfassenden Oberfläche angeordnet, sondern mit der Bildsensoreinheit verbunden ist;
- Fig. 3:: eine weitere Variante der Vorrichtung aus Figur 1, die zusätzlich einen Musterprojektor zum Projizieren eines Musters auf die zu erfassende Oberfläche aufweist; und
- Fig. 4:: eine Variante der Vorrichtung aus Figur 1, bei der die Bildsensoreinheit eine 3D-Kamera ist.

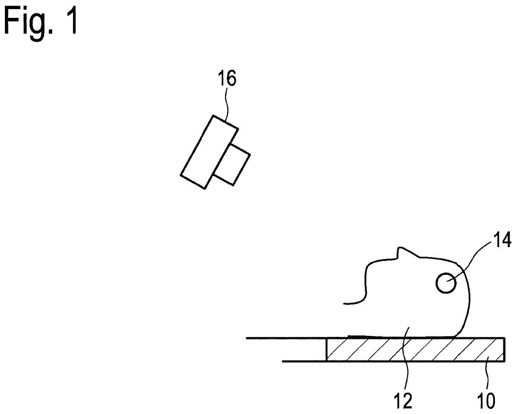

In Figur 1 ist eine Vorrichtung zum Durchführen eines Registrierverfahrens dargestellt, die ein Positionserfassungssystem 10, einen an einem Kopf 12 eines Patienten als dem zu erfassenden Körperteil angebrachten Positionssensor 14 als Referenzsensor und eine Kamera 16 als Bildsensoreinheit umfasst.

Ein zum Zwecke der Registrierung von der Kamera 16 aufgenommenes Bild zeigt sowohl das Körperteil, nämlich den Kopf 12 des Patienten, als auch den als Referenzsensor dienenden körperteilfesten Positionssensor 14 aus einer jeweiligen Perspektive. Aus mehreren Aufnahmen des Körperteils 12 kann photogrammetrisch ein Oberflächenmodell des Körperteils 12 gewonnen werden. In diesem photogrammetrisch gewonnenen Oberflächenmodell des Körperteils 12 lässt sich ebenso photogrammetrisch die Position des Positionssensors 14 bestimmen. Die Position des Positionssensors 14 ist außerdem auch über das Positionserfassungssystem 10 erfasst, so dass die photogrammetrisch bestimmte Position des Positionssensors 14 mit der vom Positionserfassungssystem 10 erfassten Position des Positionssensors 14 korreliert werden kann, so dass auch über das photogrammetrisch gewonnene Oberflächenmodell sämtliche weiteren Positionen auf der Oberfläche des Körperteils relativ zum Positionssensor 14 und damit auch in den Koordinaten des Positionserfassungssystems 10 bekannt sind.

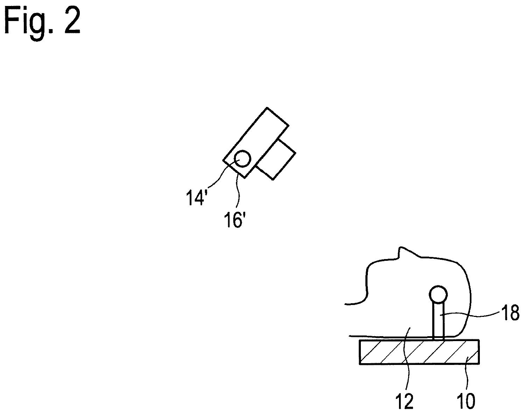

Anstelle eines körperteilfesten Positionssensors 14, wie er in Figur 1 abgebildet ist, kann auch an der Bildsensoreinheit in Form der Kamera 16' ein Positionssensor 14' vorgesehen sein. Dies ist in Figur 2 abgebildet. Auf diese Weise erfasst das Positionserfassungssystem 10 jeweils Position und Ausrichtung der Bildsensoreinheit (Kamera) 16 und kennt somit die Perspektive, aus der eine jeweilige, von der Bildsensoreinheit 16 aufgenommene Aufnahme stammt. Auch dies erlaubt es, ein genaues Oberflächenmodell aus dem von der Bildsensoreinheit 16 aufgenommenen Bildern zu generieren. In diesem Falle ist der zu erfassende Körperteil (Kopf des Patienten) 12 vorzugsweise möglichst unbeweglich mit der Positionssensoreinheit 10 verbunden. Figur 2 zeigt entsprechende Halterungen 18 für den Kopf 12 des Patienten, die mit dem Positionserfassungssystem 10 verbunden sind.

Grundsätzlich können die von der Bildsensoreinheit 16 (Kamera 16) aufgenommenen Aufnahmen bei vorhandenem Licht erfolgen. Allerdings kann in diesem Falle die Extraktion einzelner Geometrie- und Formmerkmale der fotografierten Oberfläche (beispielsweise der Oberfläche des Körperteils 12) im Einzelfall schwierig sein. Um hier zu aussagekräftigeren Aufnahmen zu kommen, kann vorteilhafterweise ein Musterprojektor 20 vorgesehen sein, der auf die Oberfläche des Körperteils 12 ein Muster 22 projiziert, das durch die dreidimensionale Gestalt der Oberfläche des Körperteils 12 jeweils perspektivisch verzerrt wird. Beispielsweise sind zur Erfassung der Topographie von Oberflächen Musterprojektionen in Form von Streifenprojektionen bekannt.

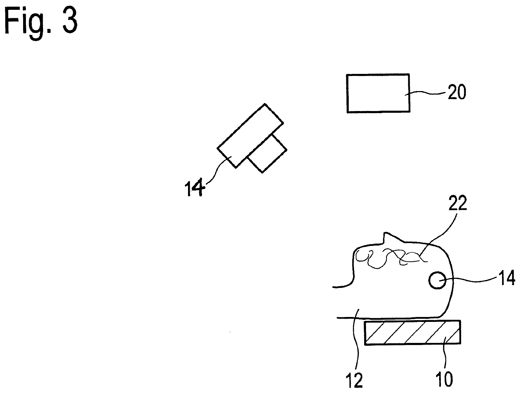

Figur 3 zeigt eine derartige Vorrichtung mit einem Musterprojektor 20.

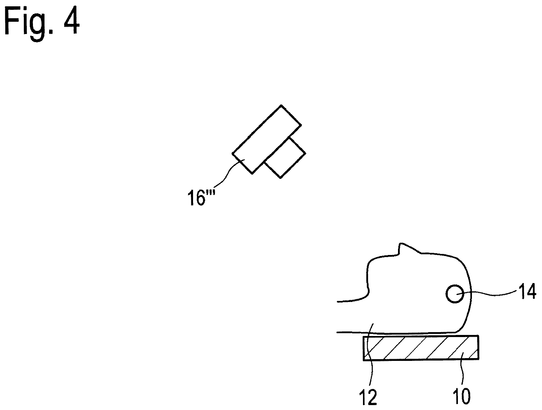

Eine Vorrichtung, bei der bereits jede einzelne Aufnahme der Bildsensoreinheit 16'" Informationen zur dreidimensionalen Oberflächengestalt des Körperteils 12 liefert, kann mit einer Bildsensoreinheit 16'" in Form einer 3-D-Kamera verwirklicht werden, wie sie in Figur 4 abgebildet ist. Derartige 3-D-Kameras 16'" sind grundsätzlich bekannt und nehmen eine dreidimensionale Oberflächenstruktur, beispielsweise nach dem time-of-flight-System auf, bei dem die Laufzeit oder die Phasenlage von Beleuchtungsimpulsen erfasst wird, die von einer entsprechenden Lichtquelle der 3-Kamera 16'" ausgehen und von der Oberfläche des Körperteils 12 reflektiert werden. Da die einzelnen Punkte der Oberfläche des Körperteils 12 einen unterschiedlichen Abstand zur Lichtquelle der Bildsensoreinheit 16'" und auch zu deren Bildsensor haben, ergeben sich unterschiedlich lange Impulslaufzeiten zwischen Lichtquelle und Bildsensor. Diese Impulslaufzeiten (oder Phasenverschiebungen) enthalten die Information über den Abstand zwischen Bildsensoreinheit 16'" und einem jeweiligen Punkt auf der Oberfläche des Körperteils 12.

In allen Fällen ist die Vorrichtung so ausgebildet, dass sie ein Oberflächenmodell mit einer Genauigkeit von 1 mm oder weniger, vorzugsweise von genauer als 0,25 mm liefert. Der Arbeitsraum der Vorrichtung bemisst sich in allen Dimensionen in einigen 10-cm, beispielsweise zwischen 200 mm x 200 mm x 200 mm und 500 mm x 500 mm x 500 mm.

## Patentansprüche

1. Registrierverfahren zum Erfassen der Position und Ausrichtung eines Objekts oder Körperteils (12) in Bezug auf ein elektromagnetisches Positionserfassungssystems (10), wobei das Verfahren die folgenden Schritte umfasst:
- Anordnen eines elektromagnetischen Referenzsensors (14) an dem Objekt oder Körperteil derart, dass der Referenzsensor optisch von einer Bildsensoreinheit erfasst werden kann, und zwar so, dass seine Relativposition zu einer zu erfassenden Oberfläche des Objekts oder Körperteils aus optisch erfassten Informationen ermittelbar ist,
- Erfassen der Position des Referenzsensors mittels des elektromagnetischen Positionserfassungssystems,
- fotogrammetrisches Erfassen einer Oberfläche des Objektes oder Körperteils und des Referenzsensors mittels einer optischen monofokalen Bildsensensoreinheit (16),
- Erzeugen eines Oberflächenmodells des Objektes oder Köperteils und des Referenzsensors, basierend auf diesen fotogrammetrisch erfassten Informationen,
- Bestimmen der Position des Referenzsensors in dem Oberflächenmodell,
- Zuordnen des Oberflächenmodells zu einem Koordinatensystem des elektromagnetischen Positionserfassungssystems anhand von der bestimmten Position des Referenzsensors in dem Oberflächenmodell und der mittels des elektromagnetischen Positionserfassungssystems erfassten Position des Referenzsensors.

2. Registrierverfahren gemäß Anspruch 1, **dadurch gekennzeichnet, dass** das fotogrammetrische Erfassen einer Oberfläche des Objektes oder Körperteils mittels einer optischen, monofokalen Bildsensoreinheit erfolgt, die mit einem Positionssensor verbunden ist oder einen solchen aufweist, dessen Position mittels des Positionserfassungssystems erfassbar ist.

3. Registrierverfahren gemäß Anspruch 2, **dadurch gekennzeichnet, dass** die Bildsensoreinheit während des fotogrammetrischen Erfassens einer Oberfläche des Objektes oder Körperteils bewegt und eine jeweilige Lage und Ausrichtung der Bildsensoreinheit mittels des Positionssensors an der Bildsensoreinheit erfasst wird.

4. Registrierverfahren nach einem der Ansprüche 1 bis 3, **dadurch gekennzeichnet, dass** für das fotogrammetrische Erfassen einer Oberfläche des Objektes oder Körperteils ein Muster auf die zu erfassende Oberfläche projiziert wird.

5. Registrierverfahren nach Anspruch 4, **dadurch gekennzeichnet, dass** das Projizieren des Musters mittels eines Musterprojektors erfolgt, der starr mit der Bildsensoreinheit verbunden ist.

6. Registrierverfahren nach Anspruch 4 oder 5, **dadurch gekennzeichnet, dass** das Projizieren des Musters mittels eines Musterprojektors erfolgt, der starr mit einem Positionssensor verbunden ist.

7. Registrierverfahren nach einem der Ansprüche 4 bis 6, **dadurch gekennzeichnet, dass** das Muster mit Infrarotlicht projiziert wird und die Bildsensoreinheit einen infrarot-empfindlichen Bildsensor aufweist.

8. Registrierverfahren nach einem der Ansprüche 1 bis 7, **dadurch gekennzeichnet, dass** als Bildsensoreinheit eine 3D-Kamera verwendet wird.

9. Registrierverfahren nach einem der Ansprüche 1 bis 8, **dadurch gekennzeichnet, dass** mehrere Positionssensoren an unterschiedlichen Orten verwendet werden oder ein beweglicher Positionssensor verwendet wird, der bei dem fotogrammetrischen Erfassen einer Oberfläche des Objektes oder Körperteils bewegt wird und deren bzw. dessen Position ebenfalls fotogrammetrisch erfasst wird, um Verzerrungen eines elektromagnetischen Wechselfeldes eines Positionserfassungssystems zu bestimmen.

10. Vorrichtung zum Durchführen eines Registrierverfahrens zum Erfassen der Position und Ausrichtung eines Objekts oder Körperteils (12) in Bezug auf ein elektromagnetisches Positionserfassungssystem (10), wobei die Vorrichtung aufweist:
- ein elektromagnetisches Positionserfassungssystem (10),
- wenigstens einen Positionssensor (14) als Referenzsensor, dessen Position und Ausrichtung mittels des elektromagnetischen Positionserfassungssystems (10) erfassbar ist, wobei der Positionssensor (14) mit einer zu erfassenden Oberfläche zu verbinden ist, und
- wenigstens eine optische monofokale Bildsensoreinheit (16), die ausgebildet ist, eine Oberfläche des Objektes oder Körperteils und des Referenzsensors fotogrammetrisch zu erfassen, wobei die Vorrichtung ausgebildet ist, ein Oberflächenmodell des Objektes oder Köperteils (12) und des Referenzsensors basierend auf diesen fotogrammetrisch erfassten Informationen zu erzeugen,
**dadurch gekennzeichnet, dass** die Vorrichtung ausgebildet ist, eine Position des Referenzsensors in dem Oberflächenmodell zu bestimmen und das Oberflächenmodell zu einem Koordinatensystem des elektromagnetischen Positionserfassungssystems (10) anhand von der bestimmten Position des Referenzsensors in dem Oberflächenmodell und der mittels des elektromagnetischen Positionserfassungssystems (10) erfassten Position des Referenzsensors zuzuordnen.

11. Vorrichtung gemäß Anspruch 10, **dadurch gekennzeichnet, dass** die Vorrichtung zusätzlich einen Musterprojektor (20) aufweist, der mit der Bildsensoreinheit (16) und/oder einem Positionssensor (14) verbunden ist und/oder einen solchen aufweist.

12. Vorrichtung gemäß Anspruch 10, **dadurch gekennzeichnet, dass** die Bildsensoreinheit (16) eine 3D-Kamera (16"') ist.

## Claims

1. A registration method for detecting the position and alignment of an object or body part (12) in relation to an electromagnetic position detection system (10), wherein the method comprises the following steps:
- arranging an electromagnetic reference sensor (14) on the object or body part such that the reference sensor can be detected optically by the image sensor unit in such way that the relative position thereof in relation to the object's or body part's surface to be detected can be established from the optically captured information,
- detecting the position of the reference sensor by means of the electromagnetic position detection system,
- photogrammetrically capturing a surface of the object or body part and of the reference sensor by means of an optical, monofocal image sensor unit (16),
- producing a surface model of the object or body part and of the reference sensor based on the photogrammetrically captured information,
- determining the position of the reference sensor in the surface model,
- correlating the surface model with a coordinate system of the electromagnetic position detection system on the basis of the determined position of the reference sensor in the surface model and on the basis of the position of the reference sensor detected by means of the electromagnetic position detection system.

2. The registration method as claimed in claim 1, **characterized in that** the photogrammetric capturing of a surface of the object or body part is performed by means of an optical, monofocal image sensor unit which is connected to a position sensor or comprises the latter, the position of which position sensor is detectable by means of the position detection system.

3. The registration method as claimed in claim 2, **characterized in that** the image sensor unit is moved during the photogrammetric capturing of a surface of the object or body part and a respective location and alignment of the image sensor unit is detected by means of the position sensor on the image sensor unit.

4. The registration method as claimed in one of claims 1 to 3, **characterized in that** a pattern is projected onto the surface to be captured for the purpose of photogrammetric capturing of a surface of the object or body part.

5. The registration method as claimed in claim 4, **characterized in that** the pattern is projected by means of a pattern projector which is rigidly connected to the image sensor unit.

6. The registration method as claimed in claim 4 or 5, **characterized in that** the pattern is projected by means of a pattern projector which is rigidly connected to a position sensor.

7. The registration method as claimed in one of claims 4 to 6, **characterized in that** the pattern is projected using infrared light and the image sensor unit comprises an infrared-sensitive image sensor.

8. The registration method as claimed in one of claims 1 to 7, **characterized in that** a 3-D camera is used as an image sensor unit.

9. The registration method as claimed in one of claims 1 to 8, **characterized in that** use is made of a plurality of position sensors at different places or of a movable position sensor which is moved during the photogrammetric capturing of a surface of the object or body part and the position of which sensor or sensors is likewise detected photogrammetrically in order to determine distortions of an alternating electromagnetic field of a position detection system.

10. A device for performing a registration method for detecting the position and alignment of an object or body part (12) in relation to an electromagnetic position detection system (10), wherein the device comprises:
- an electromagnetic position detection system (10),
- at least one position sensor (14) as a reference sensor, the position and alignment of which is detectable by means of the electromagnetic position detection system (10), wherein the position sensor (14) can be attached to a surface to be captured, and
- at least one optical, monofocal image sensor unit (16) that is configured to capture a surface of an object or a body part and of the reference sensor photogrammetrically, wherein the device is configured to generate a surface model of the object or body part (12) and of the reference sensor based on the photogrammetrically captured information,
**characterized in that** the device is configured to determine a position of the reference sensor in the surface model and to correlate the surface model with a coordinate system of the electromagnetic position detection system (10) on the basis of the determined position of the reference sensor in the surface model and on the basis of the position of the reference sensor detected by means of the electromagnetic position detection system (10).

11. The device as claimed in claim 10, **characterized in that** the device additionally comprises a pattern projector (20) which is connected to the image sensor unit (16) and/or a position sensor (14) and/or comprises one of the latter.

12. The device as claimed in claim 10, **characterized in that** the image sensor unit (16) is a 3-D camera (16"').

## Revendications

1. Procédé d'enregistrement pour détecter la position et l'orientation d'un objet ou d'une partie (12) du corps par rapport à un système (10) électromagnétique de détection de position, le procédé comprenant les stades suivants :
- mise d'un capteur (14) électromagnétique de référence sur l'objet ou la partie du corps, de manière à ce que le capteur de référence puisse être détecté optiquement par une unité de capteur d'image et cela de manière à ce que sa position relative, par rapport à une surface à détecter de l'objet ou de la partie du corps, puisse être déterminée à partir d'informations détectées de façon optique,
- détection de la position du capteur de référence au moyen du système électromagnétique de détection de position,
- détection photogrammétrique d'une surface de l'objet ou de la partie du corps et du capteur de référence au moyen d'une unité (16) de capteur d'image optique monofocale,
- production d'un modèle de surface de l'objet ou de la partie du corps et du capteur de référence sur la base de ces informations détectées photogrammétriquement,
- détermination de la position du capteur de référence dans le modèle de surface,
- association du modèle de surface à un système de coordonnées du système électromagnétique de détection de position à l'aide de la position déterminée du capteur de référence dans le modèle de surface et de la position du capteur de référence détectée au moyen du système électromagnétique de détection de position.

2. Procédé d'enregistrement suivant la revendication 1, **caractérisé en ce que** la détection photogrammétrique d'une surface de l'objet ou d'une partie du corps s'effectue au moyen d'une unité de capteur d'image optique monofocale, qui est reliée à un capteur de position ou qui en a un tel que sa position puisse être détectée au moyen du système de détection de position.

3. Procédé d'enregistrement suivant la revendication 2, **caractérisé en ce que**, pendant la détection photogrammétrique d'une surface de l'objet ou de la partie du corps, on déplace l'unité de capteur d'image et on détecte, sur l'unité de capteur d'image, une position et une orientation respective de l'unité de capteur d'image au moyen du capteur de position.

4. Procédé d'enregistrement suivant l'une des revendications 1 à 3, **caractérisé en ce que**, pour la détection photogrammétrique d'une surface de l'objet ou d'une partie du corps, on projette un modèle sur la surface à détecter.

5. Procédé d'enregistrement suivant la revendication 4, **caractérisé en ce que** l'on effectue la projection du modèle au moyen d'un projecteur de modèle, qui est relié rigidement à l'unité de capteur d'image.

6. Procédé d'enregistrement suivant la revendication 4 ou 5, **caractérisé en ce que** l'on effectue la projection du modèle au moyen d'un projecteur de modèle, qui est relié rigidement à un capteur de position.

7. Procédé d'enregistrement suivant l'une des revendications 4 à 6, **caractérisé en ce que** l'on projette le modèle par de la lumière infrarouge et l'unité de capteur d'image a un capteur d'image sensible à l'infrarouge.

8. Procédé d'enregistrement suivant l'une des revendications 1 à 7, **caractérisé en ce que** l'on utilise, comme unité de capteur d'image, une caméra 3D.

9. Procédé d'enregistrement suivant l'une des revendications 1 à 8, **caractérisé en ce que** l'on utilise plusieurs capteurs de position en des emplacements différents ou on utilise un capteur de position mobile que l'on déplace lors de la détection photogrammétrique d'une surface de l'objet ou d'une partie du corps et dont l'on détecte également photogrammétriquement la ou les positions pour déterminer des distorsions d'un champ électromagnétique alternatif d'un système de détection de position.

10. Dispositif pour effectuer un procédé d'enregistrement afin de détecter la position et l'orientation d'un objet ou d'une partie (12) du corps par rapport à un système (10) électromagnétique de détection de position, le dispositif ayant:
- un système (10) électromagnétique de détection de position,
- au moins un capteur (14) de position, comme capteur de référence, dont la position et l'orientation peuvent être détectées au moyen du système (10) électromagnétique de détection de position, le capteur (14) de position pouvant être relié à une surface à détecter et
- au moins une unité (16) de capteur d'image optique monofocale constituée pour détecter photogrammétriquement une surface de l'objet ou de la partie du corps et du capteur de référence, le dispositif étant constitué pour produire un modèle de surface de l'objet ou de la partie (12) du corps et du capteur de référence sur la base de ces informations détectées photogrammétriquement,
**caractérisé en ce que** le dispositif est constitué pour déterminer une position du capteur de référence dans le modèle de surface et pour associer le modèle de surface à un système de coordonnées du système (10) électromagnétique de détection de position à l'aide de la position déterminée du capteur de référence dans le modèle de surface et de la position du capteur de référence détectée au moyen du système (10) électromagnétique de détection de position.

11. Dispositif suivant la revendication 10, **caractérisé en ce que** le dispositif a, en outre, un projecteur (20) de modèle, qui est relié à l'unité (16) de capteur d'image et/ou à un capteur (14) de position et/ou qui en a un.

12. Dispositif suivant la revendication 10, **caractérisé en ce que** l'unité (16) de capteur d'image est une caméra en 3D (16'").
